# EUROPEAN PATENT APPLICATION

(11) **EP 2 959 901 A1**
(43) Date of publication of application: **30.12.2015**
(21) Application number: 15173049.6
(22) Date of filing: 22.06.2015
(51) Int. Cl.: A61K 31/439, A61K 9/28, A61K 31/7056, A61P 31/14

(54) **PHARMACEUTICAL COMBINATIONS OF SOFOSBUVIR AND RIBAVIRIN**

(30) Priority: 23.06.2014 TR 201407272
(71) Applicant: Sanovel Ilac Sanayi ve Ticaret A.S., 34460 Istanbul (TR)
(72) Inventor: CIFTER, Ümit, 34460 Istanbul (TR); TÜRKYILMAZ, Ali, 34460 Istanbul (TR); ERDEM, Yelda, 34460 Istanbul (TR); UCAR, Ezgi, 34460 Istanbul (TR); GÖKCEK, Sevgi, 34460 Istanbul (TR)
(74) Representative: Sevinç, Erkan

(57) **Abstract**

This invention is a novel pharmaceutical composition comprising sofosbuvir and ribavirin and at least one pharmaceutically acceptable excipient for use in the treatment of hepatitis C virus infections, chronic hepatitis C (CHC), hepatocellular carcinoma or patients with end-stage liver disease awaiting liver transplantation.

## Description

### Field of Invention

This invention is a novel pharmaceutical composition comprising sofosbuvir and ribavirin and at least one pharmaceutically acceptable excipient.

More specifically, this invention relates to pharmaceutical composition comprising sofosbuvir and ribavirin and at least one pharmaceutically acceptable excipient for use in the treatment of hepatitis C virus infections, chronic hepatitis C (CHC), hepatocellular carcinoma or patients with end-stage liver disease awaiting liver transplantation

### Background of Invention

Chronic infection with hepatitis C virus (HCV) affects more than 170 million people worldwide and is a leading cause of anticipated liver-related death due to the development of cirrhosis and its complications. Hepatitis C virus (HCV) infection is a major health problem that leads to chronic liver disease, such as cirrhosis and hepatocellular carcinoma, in a substantial number of infected individuals, estimated by the World Health Organization to be about 3% of the world's population. An estimated 150-180 million individuals are chronically infected with HCV worldwide, with 3 to 4 million people infected each year. Once infected, about 20% of people clear the virus, but the rest can harbor HCV for the rest of their lives. Ten to twenty percent of chronically infected individuals eventually develop liver-destroying cirrhosis or cancer.

Presently there are numerous antiviral drugs which are widely available. In the last 10 years, standard of care anti-HCV treatment has been founded on the combination of Peginterferon (Peg-IFN) plus ribavirin (RBV), whose main disadvantages were suboptimal rates of sustained virological response (SVR) in difficult-to-treat patients (HCV genotype 1-4, advanced liver fibrosis) and, most of all, side effects profile resulting in poor tolerability and treatment contraindication in some patient subsets (decompensated liver disease and autoimmune disorders).2 The recent availability of culture cell models provided deeper insight in understanding HCV life cycle and was the basis for the development of new drugs targeting non-structural HCV proteins involved in viral replication process, such as nonstructural protein 3 (NS3) and nonstructural protein 5A/B (NS5A/B). Direct-acting antivirals (DAAs) promised to open a new era in treating chronic HCV infection by increasing SVR rates, providing shortened and simplified regimens while also minimizing treatment-related side effects.

Sofosbuvir is a hepatitis C virus (HCV) nucleotide analog nonstructural protein 5B (NS5B) polymerase inhibitor indicated for the treatment of chronic hepatitis C (CHC) infection as a component of a combination. Sofosbuvir efficacy has been established in subjects with HCV genotype 1, 2, 3 or 4 infection, including those with hepatocellular carcinoma meeting Milan criteria (awaiting liver transplantation) and those with HCV/HIV-1 co-infection.

SOVALDI® is the brand name for sofosbuvir, a nucleotide analog inhibitor of HCV NS5B polymerase. The IUPAC name for sofosbuvir is (S)-Isopropyl 2-((S)-(((2R,3R,4R,5R)-5-(2,4-dioxo3,4-dihydropyrimidin-1 (2H)-yl)-4-fluoro-3-hydroxy-4-methyltetrahydrofuran-2yl)methoxy)-(phenoxy)phosphorylamino)propanoate. It has a molecular formula of C22H29FN3O9P and a molecular weight of 529.45. It has the following structural Formula I given below:

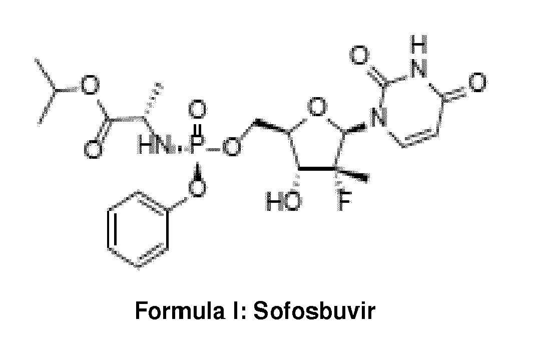

Sofosbuvir is a white to off-white crystalline solid with a solubility of ≥ 2 mg/mL across the pH range of 2-7.7 at 37°C and is slightly soluble in water. It is not metabolized by Cytochromes P450 (CYP).

In prior art, EP2203462 (B1) discloses the compound of sofosbuvir. EP2432792 (A1) discloses a process for preparing nucleoside phosphoramidates and their use as agents for treating viral diseases. EP2552933 (A1) discloses crystalline or crystal-like form of sofosbuvir.

Ribavirin is a nucleoside analogue (purine analogue) with antiviral activity. COPEGUS® and REBETOL® are the brand names for ribavirin, The chemical name of ribavirin is 1-β-Dribofuranosyl-1H-1,2,4-triazole-3-carboxamide and has the following structural Formula II given below:

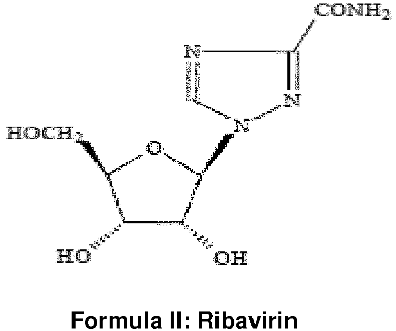

The empirical formula of ribavirin is C8H12N4O5 and the molecular weight is 242.21 Ribavirin is a white to off-white, crystalline powder. It is freely soluble in water and slightly soluble in anhydrous alcohol.

In prior art, US3798209 (A) discloses the compound of ribavirin. EP0093401 (B1) discloses a process for preparing ribavirin. EP0643970 (B1) discloses use of ribavirin in the medical treatment of viral diseases in humans.

However, clinical trials have shown that ribavirin alone can normalize W201 alanine aminotransferase (ALT) levels transiently during the course of treatment in some patients with chronic hepatitis C (CHC) infections. These studies have reported that ribavirin alone did not reduce HCV RNA levels during or after therapy and did not produce any sustained virologic response.

It is well known that drugs used in the same therapeutic area or even for treating the same indication cannot always be combined a priori with the expectation of at least additive therapeutic effects. The scientific literature is full of examples wherein compounds of different classes, which are used to treat the same indications, cannot be combined into safe and efficacious dosage forms thereby resulting in incompatible drug combinations. No pharmaceutical composition has been produced until today, which contains a combination of sofosbuvir and ribavirin. Even if some medicaments comprising either of these active agents have been administered concomitantly in practice, this fact requires the patients to carry more than one drug and causes application-related difficulties. Additionally, administering and formulating a combination, in place of the individual use of each active agent, may provide improved treatment features.

As a result, based on said drawbacks, a novelty is required in the art of pharmaceutical compositions having therapeutic effects against hepatitis C virus infections

### Detailed description of the invention

The present invention provides composition which comprises sofosbuvir and ribavirin and at least one pharmaceutically acceptable excipient. This composition with synergistic action of sofosbuvir and ribavirin eliminating all before said problems and bringing additional advantages to the relevant prior art.

Another object of the present invention is to obtain a composition with synergistic effect for use in the treatment of viral infections and preferably hepatitis C virus infections.

Another object of the present invention is to obtain a composition with synergistic action having hepatitis C virus infections, chronic hepatitis C (CHC), hepatocellular carcinoma or patients with end-stage liver disease awaiting liver transplantation treating activity.

A further object of the present invention is to obtain a combination composition having a desired level of compatibility.

With the present invention, a combination composition with synergistic action is surprisingly obtained, which has therapeutic effects against hepatitis C virus infections. Under normal conditions, the action of ribavirin against hepatitis C virus infections is slow. The action time, however, is reduced with the present invention.

Drugs of different action mechanisms can be combined. It is not possible, however, to state that a combination of drugs having different action mechanisms, but showing actions on similar targets, will have absolutely positive effects.

The term synergistic means that when drugs are administered together, a combined action is obtained which is higher than the individual actions of the respective drugs when they are used separately. On the other hand, using a lower dose of each drug to be combined according to the present invention will reduce the total dosage. Put differently, the dosages have not to be relatively less in all cases, but the drugs can be dosed less frequently or this may be beneficial in reducing the recurrence rate of side effects. These are advantageous in terms of patients to be treated.

The preferred dosages of active agents included to the pharmaceutical combination according to the present invention are therapeutically active dosages, and particularly correspond to the dosage of those which are commercially available. Therapeutically active amount not only includes therapeutic doses, but also preventive/prophylactic doses.

In one embodiment of this invention, the pharmaceutical compositions comprise the active agents in an amount ranging from 0.1% to 90%. The pharmaceutical composition comprises sofosbuvir in an amount of between 50 and 1500 mg and ribavirin in an amount of between 50 and 2000 mg.

Another embodiment of this invention, the weight ratio of sofosbuvir to ribavirin is in the range of 1:1, 1:2, 2:1, 2:3, 1:3, 3:1, 3:2, 1:4, 4:1, 1:5, 5:1, 1:6 or 6:1 (w/w) and preferably it is in the range of 1:1 to 1:6 (w/w).

Another embodiment of this invention, the therapeutically active amount of sofosbuvir and ribavirin is administrated once a day (QD) or twice a day (BID) or three times a day (TID) and dosage regimen preferably is twice a day (BID) for 6 weeks to 52 weeks.

The methods and formulations provided herein can be administered to any subject in need of therapy including, without limitation, humans or animals.

In one embodiment, these pharmaceutical combinations are administrated oral, parenteral, intranasal, sublingual, transdermal, transmucosal, ophthalmic, intravenous, pulmonary, intramuscular or rectal administration, and preferably for oral administration.

Another embodiment of this invention, the pharmaceutical composition is in the form of solid, liquid or semisolid dosage form.

According to this embodiment of the invention, said pharmaceutical composition may be formulated as dosage forms comprise tablets including compressed tablets comprising compressed tablets, coated or uncoated tablets, multilayer tablets, buccal tablets, sublingual tablets, effervescent tablets, immediate release tablets, modified release tablets, film-coated tablets, orally disintegrating tablets, gastric disintegrating tablets, effervescent compositions, pills, capsules, hard or soft gelatin capsules, powders, mini tablets, pellets, coated bead systems, granules, microspheres, ion exchange resin systems, sterile solutions or suspensions, steril ocular solutions, aerosols, sprays, drops, ampoules, suppositories, ocular systems, parenteral systems, creams, gels, ointments, dragees, sachets, films, orally administrable films, solutions, solids, elixirs, tinctures, suspensions, syrups, colloidal dispersions, dispersions, emulsions and thereof.

The pharmaceutical composition of the invention is formulated preferably in the form of tablet or capsule or multilayer tablet.

In one embodiment, the pharmaceutical composition of the invention is for use in the treatment of viral infections and preferably hepatitis C virus infections, chronic hepatitis C (CHC), hepatocellular carcinoma or patients with end-stage liver disease awaiting liver transplantation treating activity.

According to the challenges mentioned above the selection of the excipients thus very important. According to this embodiment, one or more pharmaceutically acceptable excipient is selected from the group comprising buffering agents, stabilizers, antioxidants, binders, diluents, dispersing agents, lubricants, glidants, disintegrants, plasticizers, preservatives, sweeteners, flavoring agents, coloring agents or mixtures thereof.

Suitable buffering agents may comprise but not limited to alkali metal citrate, citric acid/sodium citrate, tartaric acid, fumaric acid, sorbic acid, citric acid, succinic acid, adipic acid, ascorbic acid, glutaric acid, potassium hydrogen tartrate, sodium hydrogen tartrate, potassium hydrogen phthalate, sodium hydrogen phthalate, potassium dihydrogen phosphate, sodium dihydrogen phosphate, disodium hydrogen phosphate, hydrochloric acid/sodium hydroxide or mixtures thereof, and preferably citric acid, fumaric acid, ascorbic acid, sodium dihydrogen phosphate, glycin , glutamic acid or mixtures thereof.

Suitable stabilizers may comprise but not limited to citric acid, fumaric acid, tartaric acid, sodium citrate, sodium benzoate, sodium dihydrogen phosphate, calcium carbonate, magnesium carbonate, arginine, lysine, meglamine, ascorbic acid, gallic acid esters or the mixtures thereof, and preferably, citric acid, fumaric acid, arginine or mixtures thereof.

Suitable antioxidants may comprise but not limited to alpha tocopherol, ascorbic acid, ascorbyl palmitate, butylhydroxyanisole (BHA), butylhydroxytoluene (BHT), erythorbic acid, monothioglycerol, potassium metabisulfite, propyl gallate, sodium ascorbate, sodium metabisulfite, sodium sulfite, thymol or mixtures thereof.

Suitable binders may include but not limited to polyvinylpyrrolidone, polyethylene glycol, polyvinyl alcohol, starch, pregelatinized starch, glucose, glucose syrup, natural gums, sucrose, sodium alginate, cellulose derivatives such as hydroxypropyl methyl cellulose, hydroxypropyl cellulose, carboxy methyl cellulose, methyl cellulose, gelatin, carrageenan, guar gum, carbomer, polymethacrylates, methacrylate polymers, collagens, proteins like gelatin, agar, alginate, alginic acid, xanthan gum, hyaluronic acid, pectin, polysaccharides, carbomer, poloxamer, polyacrylamide, aluminium hydroxide, laponit, bentonit, polyoxyethylene-alkyl ether, polydextrose, polyethylene oxide or mixtures thereof.

Suitable diluents may comprise but not limited to microcrystalline cellulose, mannitol, spray-dried mannitol, lactose, lactose monohydrate, starch, dextrose, sucrose, fructose, maltose, sorbitol, xylitol, inositol, kaolin, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

Suitable dispersing agents may comprise but not limited to calcium silicate, magnesium aluminum silicate or mixtures thereof.

Suitable lubricants may comprise but not limited to magnesium stearate, colloidal silicon dioxide, calcium stearate, zinc stearate, talc, waxes, boric acid, hydrogenated vegetable oil, sodium chlorate, magnesium lauryl sulfate, sodium oleate, sodium acetate, sodium benzoate, polyethylene glycol, stearic acid, fatty acid, fumaric acid, glyseryl palmito sulphate, sodium stearyl fumarate, sodium lauryl sulphate or mixtures thereof.

Suitable glidants may comprise but not limited to talc, aluminium silicate, colloidal silica, starch or mixtures thereof.

Suitable disintegrants may comprise but not limited to cross-linked polyvinil pyrrolidone (crospovidone), povidone, cross-linked carboxymethyl cellulose (croscarmellose sodium), low-substituted hydroxypropyl cellulose, pregelatinized starch, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate, sodium lauryl sulphate or mixtures thereof.

Suitable plasticizers may comprise but not limited to polyethylene glycols of different molecular weights, propylene glycol or mixtures thereof.

Suitable preservatives may comprise but not limited to methyl paraben, propyl paraben and their salts (such as sodium, potassium), sodium benzoate, citric acid, benzoic acid, butylated hydroxytoluene or butylated hydroxyanisole, m-cresol, phenol or mixtures thereof.

Suitable sweeteners may comprise but not limited to aspartame, potassium acesulfame, sodium saccharinate, neohesperidine dihydrochalcone, sucralose, saccharin, sugars such as sucrose, glucose, lactose, fructose or sugar alcohols such as mannitol, sorbitol, xylitol, erythritol or mixtures thereof.

Suitable flavoring agents may comprise but not limited to menthol, peppermint, cinnamon, chocolate, vanillin or fruit essences such as cherry, orange, strawberry, grape, black currant, raspberry, banana, red fruits, wild berries or mixtures thereof. Suitable coloring agents may comprise but not limited to ferric oxide, titanium dioxide, Food, Drug & Cosmetic (FD&C) dyes (such as; FD&C blue, FD&C green, FD&C red, FD&C yellow, FD&C lakes), poncau, indigo Drug & Cosmetic (D&C) blue, indigotine FD&C blue, carmoisine indigotine (indigo Carmine); iron oxides (such as; iron oxide red, yellow, black), quinoline yellow, flaming red, carmine, carmoisine, sunset yellow or mixtures thereof.

In one embodiment of the invention, the pharmaceutical composition may comprise optionally an inert layer between the two molecules wherein the inert layer is selected from the group comprises starch, lactose, sugar alcohol (D-mannitol, erythritol, etc.), lowsubstituted hydroxypropyl cellulose, hydroxypropyl cellulose, hydroxypropyl methylcellulose, polyvinylpyrrolidone, polyvinyl alcohol, methylcellulose, hydroxyethyl methylcellulose or mixtures thereof.

In one embodiment of the invention, the pharmaceutical composition may comprise optionally a coating wherein the coating material is selected from the group comprising iron oxide yellow, polyvinyl alcohol-polyethylene glycol copolymers (Kollicoat IR), polyvinylalcohol based films (Opadry 200), polyvinyl alcohol or copolymers or mixtures thereof (Opadry AMB), hydroxypropyl methyl cellulose (HPMC), ethyl cellulose, ethylcellulose dispersions (Surelease), Kerry-HPC, polyethylene glycol, polyvinylprolidone, polyvinylprolidone-vinyl acetate copolymer (PVP-VA) and all kinds of OpadryTM, as well as pigments, dyes, titanium dioxide, iron oxide, talc, chromatone-P, triacetin or polymethylmetacrylate copolymers (Eudragit).

According to the pharmaceutical compositions of the invention may be prepared by conventional technology well known to those skilled in the art such as direct compression, dry granulation, wet granulation. During direct compression, active agent and excipients are mixed, sieved and compressed into dosage forms. During wet granulation, the ingredients are mixed and granulated with a granulation liquid. The granulation process provides agglomerates with a desired homogeneity. The mixture is dried and sieved and optionally mixed with additional excipients. Finally, it is compressed into dosage forms. In addition, this novel pharmaceutical formulation is produced by various technologies such as fluidized bed granulation technique or extrusion/spheronization or spray drying and lyofilization.

### Examples:

### Example-1: Sofosbuvir+Ribavirin Tablet

| **Ingredients** | **% amount** |
|---|---|
| **Active Ingredients** | |
| Sofosbuvir | 5-95% |
| Ribavirin | 5-95% |

| **Inactive ingredients** | |
|---|---|
| Microcrystalline cellulose (MCC) | 15-60% |
| Croscarmellose Sodium | 0.5-5% |
| Magnesium stearate | 0.25-5% |
| Colloidal silicon dioxide | 0.1-5% |
| Mannitol | 10-90% |
| Corn starch | 3-25% |
| Pregelatinized starch | 5-20% |

| **Film Coating** | |
|---|---|
| HPMC | 1-5% |
| Ethyl cellulose | 1-3% |
| Talc | 10-25% |
| Titanium dioxide | 10-20% |
| Iron oxide | 1-2% |

The process for the preparation of film coated tablet of sofosbuvir and ribavirin including the steps of:
a) blending sofosbuvir with mannitol, microcrystalline cellulose (MCC), croscarmellose sodium, colloidal silicon dioxide, magnesium stearat.
b) Dry granulating the blend of step a);
c) sieved the blend of step b);
d) blending ribavirin with microcrystalline cellulose (MCC), corn starch, pregelatinized starch, croscarmellose sodium.
e) Wet granulating the blend of step d);
f) sieved the blend of step e);
g) blending the granules of step c) and f) with magnesium stearate and colloidal silicon dioxide
h) compressing the blend of step g) into a tablet;
i) optionally, film coating the tablet of step h).

### Example-2: Sofosbuvir+Ribavirin Pellets (Tablet or Capsule)

| **Ingredients** | **% amount** |
|---|---|
| **Sofosbuvir Pellets** | |
| Sofosbuvir | 5-95% |
| Microcrystalline cellulose (MCC) | 15-40% |
| Pregelatinized starch | 5- 50% |

| **Ribavirin Pellets** | |
|---|---|
| Ribavirin | 5-95% |
| Polyvinylpyrrolidone K30 (PVP K30) | 2-5% |
| Sugar pellets | 5-90% |

| **Inactive ingredients** | |
|---|---|
| Silicon dioxide | 0.1-0.2% |
| Magnesium stearate | 0.25-2.0% |

The process for the preparation of pellets of sofosbuvir and ribavirin including the steps of:

### Sofosbuvir Pellets:

a) blending sofosbuvir with microcrystalline cellulose (MCC) and pregelatinized starch
b) by spraying water a wet mass is formed from step a);
   pellets produced from this wet mass of step b) by extrusion/spheronization pelletizing technique

### Ribavirin Pellets:

a) blending ribavirin with PVP K30
b) by adding water a solution/dispersion is prepared from step a)
c) sugar pellets are coated with the solution/dispersion from step b);

The sofosbuvir and ribavirin pellets first mixed with silicon dioxide and then with magnesium stearate. The pellets are:
i. pressed as tablets and coated
ii. or filled into the capsules

### Example-3: Sofosbuvir+Ribavirin Multilayer Tablet

| **Ingredients** | **% amount** |
|---|---|
| **Sofosbuvir Layer** | |
| Sofosbuvir | 5-95% |
| Microcrystalline cellulose (MCC) | 15-40% |
| Croscarmellose sodium | 0.5-5% |
| Mannitol | 5-30% |

| **Inert layer** | |
|---|---|
| Microcrystalline cellulose (MCC) | 10-40% |
| Hydroxypropyl cellulose | 0.1-10% |
| Yellow iron oxide | 0.1-10% |

| **Ribavirin Layer** | |
|---|---|
| Ribavirin | 5-95% |
| Croscarmellose sodium | 0.5-5% |
| Microcrystalline cellulose (MCC) | 15-40% |
| Starch | 5-20% |

| **Other Excipients** | |
|---|---|
| Magnesium stearate | 0.1 - 5.0% |
| Colloidal silicon dioxide | 0.1 - 5% |

The process for the preparation of multilayer tablet of sofosbuvir and ribavirin including the steps of:

### Sofosbuvir Layer

a) blending sofosbuvir with microcrystalline cellulose (MCC) and croscarmellose and mannitol
b) granulating the blend of step a);
c) sieved the blend of step b);
d) blending the granules of step c) with magnesium stearate and colloidal silicon dioxide)

### Ribavirin Layer

a) blending ribavirin with microcrystalline cellulose (MCC) and croscarmellose sodium and starch
b) granulating the blend of step a);
c) sieved the blend of step b);
d) blending the granules of step c) with magnesium stearate and colloidal silicon dioxide

### Inert layer

There are two ways for the preparation of inert layer:
a) A solution/dispersion is prepared by adding microcrystalline cellulose (mcc), hydroxypropyl cellulose and yellow iron oxide as inert layer and one of the layers of Sofosbuvir or Ribavirin is coated by spraying this mixture in fluidized bed.
b) Microcrystalline cellulose (mcc), hydroxypropyl cellulose and yellow iron oxide are blended. The mixture is sieved and magnesium stearate and colloidal silicon dioxide are added.

At last, these different mixtures obtained are pressed in the form of multilayer tablets so that the inert layer is formed in the middle, i.e. forms the intermediate layer. Optionally, this multilayer tablet can be coated.

## Claims

1. A pharmaceutical composition comprising sofosbuvir and ribavirin and at least one pharmaceutically acceptable excipient.

2. The pharmaceutical composition according to claim 1, wherein sofosbuvirin an amount of between 50 and 1500 mg and ribavirin in an amount of between 50 and 2000 mg.

3. The pharmaceutical composition according to claim 2, wherein the weight ratio of sofosbuvir to ribavirin is in the range of 1:1, 1:2, 2:1, 2:3, 1:3, 3:1, 3:2, 1:4, 4:1, 1:5, 5:1, 1:6 or 6:1 (w/w) and preferably it is in the range of 1:1 to 1:6 (w/w).

4. The pharmaceutical composition according to claim 2 or 3, wherein said composition is administrated once a day or twice a day or three times a day and dosage regimen preferably is twice a day for 6 weeks to 52 weeks.

5. The pharmaceutical composition according to claim 1, wherein it is in the form of solid, liquid or semisolid dosage form.

6. The pharmaceutical composition according to claim 5, wherein the said composition as dosage form is selected from the group comprising tablets comprising compressed tablets, coated or uncoated tablets, multilayer tablets, buccal tablets, sublingual tablets, effervescent tablets, immediate release tablets, modified release tablets, film-coated tablets, orally disintegrating tablets, gastric disintegrating tablets, effervescent compositions, pills, capsules, hard or soft gelatin capsules, powders, mini tablets, pellets, coated bead systems, granules, microspheres, ion exchange resin systems, sterile solutions or suspensions, steril ocular solutions, aerosols, sprays, drops, ampoules, suppositories, ocular systems, parenteral systems, creams, gels, ointments, dragees, sachets, films, orally administrable films, solutions, solids, elixirs, tinctures, suspensions, syrups, colloidal dispersions, dispersions, emulsions and thereof.

7. The pharmaceutical composition according to claim 6, wherein said pharmaceutical composition is formulated preferably in the form of tablet or capsule or multilayer tablet.

8. The pharmaceutical composition according to claim 1, wherein the one or more pharmaceutically acceptable excipients are selected from the group comprising buffering agents, stabilizers, antioxidants, binders, diluents, dispersing agents, lubricants, glidants, disintegrants, plasticizers, preservatives, sweeteners, flavoring agents, coloring agents or mixtures thereof.

9. The pharmaceutical composition according to any preceding claims comprising;
a) 5.00 - 95.00 % by weight of sofosbuvir
b) 5.00 - 95.00 % by weight of ribavirin
c) 15.00 - 60.00 % by weight of microcrystalline cellulose
d) 0.50 - 5.00 % by weight of croscarmellose
e) 0.25 - 5.00 % by weight of magnesium stearate
f) 0.10 - 5.00 % by weight of colloidal silicon dioxide
g) 10.00 - 90.00 % by weight of mannitol
h) 3.00 - 25.00 % by weight of corn starch
i) 3.00 - 25.00 % by weight of pregelatinized starch
j) optionally film coating
i. 1.00 - 5.00 % by weight of hydroxypropyl methylcellulose
ii. 1.00 - 3.00 %by weight of ethyl cellulose
iii. 10.00 - 25.00 % by weight of talc
iv. 10.00 - 20.00 % by weight of titanium dioxide
v. 1.00 - 2.00 % by weight of iron oxide

10. The pharmaceutical composition according to any preceding claims comprising;
a) 5.00 - 95.00 % by weight of sofosbuvir
b) 15.00 - 40.00 % by weight of microcrystalline cellulose
c) 10.00 - 50.00 % by weight of pregelatinized starch
d) 5.00 - 95.00 % by weight of ribavirin
e) 2.00 - 5.00 % by weight of polyvinylpyrrolidone K30
f) 5.00 - 90.00 % by weight of sugar pellet
g) 0.10 - 0.20 % by weight of silicon dioxide
h) 0.25 - 2.00 % by weight of magnesium stearate
i) optionally film coating

11. The pharmaceutical composition according to any preceding claims comprising;
a) 5.00 - 95.00 % by weight of sofosbuvir
b) 15.00 - 40.00 % by weight of microcrystalline cellulose
c) 0.50 - 5.00 % by weight of croscarmellose sodium
d) 0.50 - 30.00 % by weight of mannitol
e) 10.00 - 40.00 % by weight of microcrystalline cellulose
f) 0.10 - 10.00 % by weight of hydroxypropyl cellulose
g) 0.10 - 10.00 % by weight of yellow iron oxide
h) 5.00 - 95.00 % by weight of ribavirin
i) 15.00 - 40.00 % by weight of microcrystalline cellulose
j) 5.00 - 20.00 % by weight of starch
k) 0.10 - 5.00 % by weight of magnesium stearate
l) 0.10 - 5.00 % by weight of silicon dioxide
m) optionally film coating

12. The pharmaceutical composition according to any preceeding claims, wherein the pharmaceutical composition is for use in the treatment of viral infections and preferably hepatitis C virus infections. chronic hepatitis C , hepatocellular carcinoma or patients with end-stage liver disease awaiting liver transplantation treating activity.
